# EUROPEAN PATENT APPLICATION

(11) **EP 4 349 258 A1**
(43) Date of publication of application: **10.04.2024**
(21) Application number: 23199988.9
(22) Date of filing: 27.09.2023
(51) Int. Cl.: A61B 5/22, A61B 5/24, H04B 5/48, H04B 5/72, H04B 5/73, H04W 4/38, H04W 4/80

(54) **WIRELESS DEVICE NETWORK TOPOLOGY**

(30) Priority: 30.09.2022 IN 202221056241
(71) Applicant: NXP B.V., 5656 AG Eindhoven (NL)
(72) Inventor: Meijer, Rinze Ida Mechtildis Peter, 5656 AG Eindhoven (NL); Rajan Kesavelu Shekar, Pramod, 5656 AG Eindhoven (NL)
(74) Representative: Miles, John Richard

(57) **Abstract**

One example discloses a first wireless network device, including: a far-field transceiver; a near-field transceiver; wherein the first wireless network device is configured to be wirelessly near-field coupled to a second wireless network device that also includes a far-field transceiver and a near-field transceiver; wherein the first wireless network device is further configured to be wirelessly near-field coupled to a set of near-field wireless network devices using only the near-field transceiver; wherein first wireless network device, the second wireless network device, and the set of near-field wireless network devices are configured to be in physical contact with a body; and wherein the first wireless network device is configured to be wirelessly far-field coupled to a third wireless network device only if the second wireless network device is not wirelessly far-field coupled to the third wireless network device.

## Description

The present specification relates to systems, methods, apparatuses, devices, articles of manufacture and instructions for wireless networking.

### SUMMARY

According to an example embodiment, a first wireless network device, comprising: a far-field transceiver; a near-field transceiver; wherein the first wireless network device is configured to be wirelessly near-field coupled to a second wireless network device that also includes a far-field transceiver and a near-field transceiver; wherein the first wireless network device is further configured to be wirelessly near-field coupled to a set of near-field wireless network devices using only the near-field transceiver; wherein first wireless network device, the second wireless network device, and the set of near-field wireless network devices are configured to be in physical contact with a body; and wherein the first wireless network device is configured to be wirelessly far-field coupled to a third wireless network device only if the second wireless network device is not wirelessly far-field coupled to the third wireless network device.

In another example embodiment, the first wireless network device, the second wireless network device, and the set of near-field wireless network devices together form a wireless near-field body area network (WBAN) according to an IEEE 802.15.6 standard.

In another example embodiment, the first wireless network device, the second wireless network device, and the third wireless network device together form a wireless far-field personal area network (WPAN) according to an IEEE 802.15.4 standard.

In another example embodiment, if the first wireless network device is far-field coupled to the third wireless network device, then the second wireless network device is blocked from being far-field coupled to the third wireless network device.

In another example embodiment, the third wireless network device is far-field coupled to only one of the wireless network devices that are in physical contact with the body.

In another example embodiment, only one of either the first wireless network device and the second wireless network device is far-field coupled to the third wireless network device at a time.

In another example embodiment, the third wireless network device communicates with all on-body devices using only the far-field coupling with either the first wireless network device or the second wireless network device.

In another example embodiment, the far-field coupling uses plane wave RF signals; and the near-field coupling uses quasi-static electric signals.

In another example embodiment, the first and second wireless network devices are on-body devices; the third wireless network devices is an off body device; if the first wireless network device is wirelessly far-field coupled to the third wireless network device then the first wireless network device is a Leader device; and if the second wireless network device is not wirelessly far-field coupled to the third wireless network device then the second wireless network device is a Follower device.

In another example embodiment, either the on-body Leader device, the on-body Follower device, or the off-body device is configured to switch the far-field coupling from the Leader device to the Follower device in response to a trigger condition.

In another example embodiment, the trigger condition occurs if the Leader device is not able to far-field couple with the off body device.

In another example embodiment, the trigger condition occurs if the Leader device consumes more power to wirelessly couple to the off-body device than the Follower device.

In another example embodiment, the trigger condition occurs if the Leader device has a lower battery energy level than the Follower device.

In another example embodiment, the trigger condition occurs if the Leader device has a lower remaining battery capacity than the Follower device.

In another example embodiment, the trigger condition occurs if the Leader device at least one of reaches an end-of life, fails, or is disabled.

In another example embodiment, the trigger condition occurs if the Leader device has a weaker signal connection to the off body device than the Follower device.

In another example embodiment, after the trigger condition and a threshold period of time the far-field coupling switches back to the Leader device from the Follower device.

In another example embodiment, either the on-body Leader device, the on-body Follower device, or the off-body device is configured to ping or poll each other to check if a far-field coupling is possible.

In another example embodiment, body includes at least: a human's body, an animal's body, a body of a living organism, a body structure of an inanimate object, a robot, a vehicle, a docking system, a physical coupling system, a station on an assembly line, an edge device, an Internet-of Things (IoT) device.

In another example embodiment, the body is in between the first wireless network device and the second wireless network device.

The above discussion is not intended to represent every example embodiment or every implementation within the scope of the current or future Claim sets. The Figures and Detailed Description that follow also exemplify various example embodiments.

Various example embodiments may be more completely understood in consideration of the following Detailed Description in connection with the accompanying Drawings.

### BRIEF DESCRIPTION OF THE DRAWINGS

Figure 1A is an example multi-point to single-point on-body WBAN plus a single-point to single-point on-body to off body WPAN.
Figure 1B is an example multi-point to single-point off body WPAN.
Figure 2 represents a first example of a wireless network topology.
Figure 3 represents a second example of the wireless network topology.
Figure 4 represents an example set of instructions for a wireless network topology initialization process.
Figure 5 represents an example set of instructions for a wireless network topology exchange of data between on-body and off-body devices when new data is available at a Leader node.
Figure 6 represents an example set of instructions for a wireless network topology exchange of data between on-body and off-body devices when new data is available at a Follower node.
Figure 7 represents an example set of instructions for a wireless network topology when new data is available at an off body device to be transmitted to a Leader node, and the data is addressed to the Leader node.
Figure 8 represents an example set of instructions for a wireless network topology when new data is available at an off body device to be transmitted to a Leader node, but the data is addressed to a Follower node.
Figure 9 represents an example set of instructions for checking connection possibility between on-body and off-body devices.

While the disclosure is amenable to various modifications and alternative forms, specifics thereof have been shown by way of example in the drawings and will be described in detail. It should be understood, however, that other embodiments, beyond the particular embodiments described, are possible as well. All modifications, equivalents, and alternative embodiments falling within the spirit and scope of the appended claims are covered as well.

### DETAILED DESCRIPTION

Wireless Body Area Networks (WBANs) (e.g. conforming to IEEE Standard 802.15.6) support on-body communications. Examples of on-body communications includes near-field communications inside and around a body, structure, device, etc.

Near-field communication can be effected using a variety of device designs, including near-field electromagnetic induction (NFEMI), where the transmitter and receiver are coupled by both magnetic (H) and electric (E) fields, near-field electric-induction (NFEI), where the transmitter and receiver are coupled by electric (E) fields, and near-field magnetic-induction (NFMI/NFC), where the transmitter and receiver are coupled by magnetic (H) fields. NFEMI, NFEI, NFMI and NFC devices communicate using non-propagating quasi-static E and/or H field signals.

Some WBANs in healthcare and medical fields are called Medical Body Area Networks (MBANs); however, continuous glucose monitoring (CGM) and insulin therapy instead make use of standard Bluetooth Low Energy wireless communication.

Wireless Personal Area Networks (WPANs) (e.g. conforming to IEEE Standard 802.15.4) support off-body communications. An example of off-body communications includes far-field communications where two or more bodies, structures, devices, etc. are separated by free-space. Far-field wireless communication is accomplished by propagating an RF plane wave through free space, and includes device designs such as RF, WiFi and BLE.

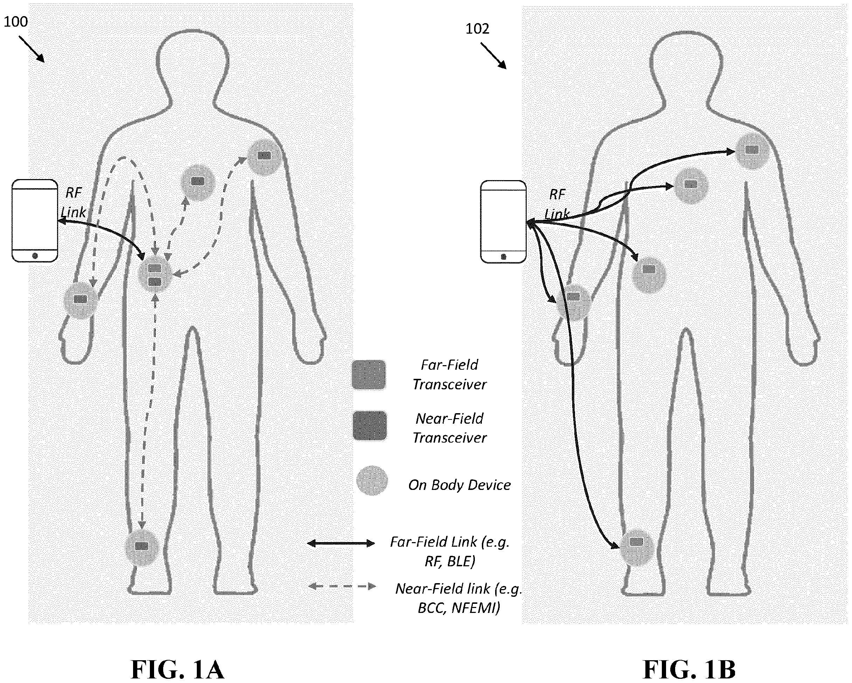

Figure 1A is an example 100 multi-point to single-point on-body WBAN plus a single-point to single-point on-body to off-body WPAN, and Figure 1B is an example 102 multi-point to single-point off-body WPAN. The single RF link in example 100 benefits interoperability, coexistence and security by separating on-body (WBAN) and off-body (WPAN) frequency spectrum and communication range, and requiring only one device to have an RF radio. The multi RF link in example 102 benefits a lower system cost as only one RF radio is needed in each networked device as shown.

Since in most medical contexts both WBANs and WPANs must be reliable/robust, secure and interoperable, the above two examples 100, 102 have several potential problems.

For example, a major problem to be resolved for medical companies concern to achieve robust and resilient RF communication for artificial pancreas use-cases, (i.e. link between continuous glucose monitoring (CGM) sensor patch, connected insulin pump and smart phone). The issue mainly occurs when users/patients are sleeping - while they lie on top of one of the body worn devices. Such scenario is a root cause of RF connection issues between CGM and insulin pump, and also RF connection towards an off-body device (e.g. smartphone) cannot be guaranteed. These link issues cause false alarms that, due to medical safety protocols, wake-up the user/patients in the middle of their sleep.

In another example, RF body shadowing effects cause link range degradation. Human body shadowing is caused by the presence of a user/patient, who can block the line-of-sight (LoS) (e.g. is in between) of a RF connection between on-body devices, as well as between on-body and off body device. The human body encompasses around 70% of water that can absorb part of the radio signal, thereby impacting the link range tremendously. Moreover, the human body can scatter the longer radio signal waves while reflecting or attenuating the shorter ones due to its conductive nature.

A further problem is a growing number of RF devices leading to occupied frequency bands and packet collisions. For instance, many of today's body-worn devices are equipped with RF transceivers i.e. Bluetooth Low Energy (BLE) to communicate between themselves as well as well off-body devices. The BLE frequency spectrum is also used by other communication technologies such as WiFi, Bluetooth, and Zigbee. There is a growing trend towards smart devices utilized for applications such as smart home, PC & Gaming, Home entertainment, wearables, hearables, smart speakers, and others. This leads to the issue that the BLE frequency spectrum can be heavily used in certain environments - causing potential link issues is such environments. Today's 2.4GHz interference profile is much worse today than few years ago, which causes mainly issues for low-latency RF communication where re-transmissions are of concern.

Now discussed is a wireless device network topology for mitigating communication link issues such as those described above that can occur between on-body and off-body devices. Some example embodiments of the wireless device network topology employed includes a first wireless network device having both a far-field transceiver and a near-field transceiver and a second wireless network device also having both a far-field transceiver and a near-field transceiver.

These first and second wireless devices are connected to both a WBAN and WPAN network and depending upon their dynamic use, assume a network roll as either a herein defined "Leader device/node" or a herein defined "Follower device/node". Herein, a wireless network "node" and a wireless network "device" are used interchangeably. Also herein, "in physical contact with" the body and "on body" are used interchangeably, and "not in physical contact with" the body and "off body" are used interchangeably.

This network topology is resistant to body-shadowing, fading and interference while also conserving power. Such robustness is achieved by keeping only one off-body (e.g. far-field) wireless link active at a time and using on-body (near-field) communication between two or more hybrid (i.e. having both near-field and far-field capabilities) nodes to shift between Leader node and Follower node roles in response to various trigger/threshold conditions.

Note, while example embodiments in the following discussion may refer to a human body, a user's body, on-body and off-body, in alternate embodiments the near-field device, body is herein broadly defined to include at least: a human's body, an animal's body, a body of a living organism, a body structure of an inanimate object, a robot, a vehicle, a docking system, a physical coupling system, a station on an assembly line, and so on.

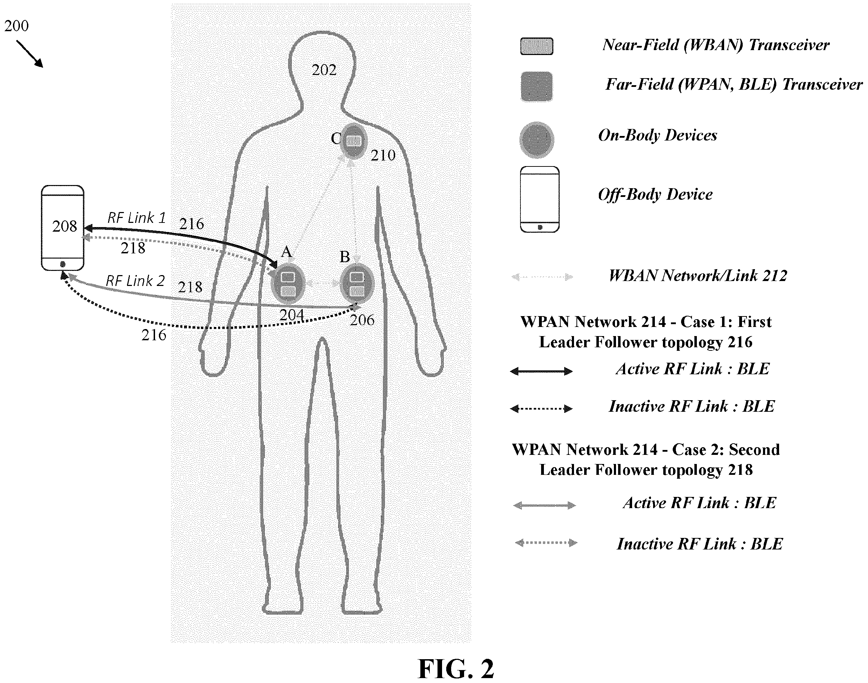

Figure 2 represents a first example 200 wireless network topology. The first example 200 wireless network topology shows a body 202 (e.g. user/patient) that is wearing two on-body wireless devices 204, 206 also referred to herein as main nodes (e.g. Node A and Node B).

A main node contains both a far-field (WPAN) transceiver (e.g. RF, BLE) and a near-field (WBAN) transceiver (e.g. NFEMI) as shown. The main nodes may in some example embodiments also include a sensor unit (e.g. CGM sensor), an actuator unit (e.g. insulin pump), a processing & control unit, a memory storage unit, and a power unit, for powering the related on-body device. The main nodes 204, 206 are capable to communicate with an off-body wireless device 208 directly via a far-field (e.g. RF) link, while also being capable of communicating to other on-body near-field wireless devices/nodes 210 via the WBAN network 212.

The off body device 208 uses longer-range far-field WPAN communication, and in various example embodiments may include: an RF transceiver (e.g. Bluetooth LE transceiver); a processing & control unit, a memory storage unit, a display unit, and a power unit, for powering the related off-body device.

In some example embodiments the main nodes 204, 206 can be mapped to a CGM sensor patch (Node A) and an insulin patch pump (Node B). In various example embodiments, the body 202 (e.g. user/patient) wears at least two main nodes to ensure robust communication with the off-body node 208, but optionally may wear more on-body nodes like 204 and 206. Additionally, the body 202 may wear one or more further nodes 210 (e.g. Node C) that contain a near-field WBAN transceiver alone. This node 210 could for example be a medical vital sign monitoring patch. This further node 210 does not communicate with the off body device 208 directly, but only indirectly via the main nodes 204, 206 that contain the far-field transmitter.

In various example embodiments, only one of the main node wireless devices 204, 206 hosts an active far-field RF link to the off body device 208 for data communication purposes. The wireless device 204 or 206 that hosts the active far-field RF link to the off body device 208 is herein defined as a Leader node. The wireless device 204 or 206 that remains inactive and does not communicate using the far-field RF link to the off body device 208 is herein defined as a Follower node.

Thus, in many example embodiments, both nodes 204, 206 do not host a far-field link to the off-body device 208 at a same time, and only RF Link 1 or RF Link 2 (as shown) is active during normal operation. Communication with the off body device 208 is performed while only making use of one active RF link between Leader node and off-body device 208. The Follower node takes over communication from the Leader node when RF link issues occur (e.g. when a sleeping patient lies on top of the Leader node).

In some example embodiments, the off body node 208 is programmed to initiate switching of the RF communication channel from a current Leader node to a Follower node (which becomes a new Leader node), such as when there are connection and/or quality issues between the off-body device and the current Leader node.

In other example embodiments, either the on-body Leader node or the on-body Follower node is programmed to initiate switching of the RF communication channel from a current Leader node to a Follower node (which becomes a new Leader node), such as when there are connection and/or quality issues between the off-body device and the current Leader node.

Some example scenarios that initiate/trigger switching of the far-field (e.g. RF) communication channel from a current Leader node to a Follower node (which then becomes a new Leader node) are now described.
Scenario 1: on-body Node A is not able to far-field pair with the off body device 208 (e.g. Mobile Device) thereby triggering Node B to be initiated to be a New Leader Node and then far-field pair with the off body device 208. This scenario can happen for example, when the user/body is sleeping on their side and blocks a line of sight between Node A and the off body device 208.
Scenario 2: In this scenario, after a Leader to Follower switch, the roles of Leader node and Follower node then automatically resume back/return to their prior Leader Follower roles after a predetermined interval and provided that successful RF Link can be reestablished.
Scenario 3: In this Scenario, the Leader and Follower roles are dependent upon each node's power state and/or power requirements. For example a node which evaluates to be a lowest power consumer for the wireless communication could be assigned as a Leader node.
Scenario 4: In this Scenario, the Leader and Follower roles are based on currently available Battery Levels and/or capacity (e.g. larger battery) at their corresponding nodes. For example a node with highest Battery level could be assigned as a Leader Node.
Scenario 5: In this Scenario, the Leader and Follower roles switch if either node is disabled, fails or expires (e.g. end of life).
Scenario 6: In this Scenario, the Leader and Follower roles are based on a comparative best reception at a time of initialization (i.e. Leader node assigned to node with a best signal strength when the WBAN network 212 and WPAN network 214 are powered on, established, etc. )

To enhance robustness of the far-field communications link, the Leader and Follower nodes are positioned at different locations on the body 202 and with sufficient physical spacing, while interacting between themselves via WBAN communications and with off body devices using WPAN communications. On-body devices communicate wirelessly with the Leader node via WBAN (short-range) communication utilizing their respective WBAN transceivers. The off-body device communicates wirelessly with the Leader node of the WBAN by means of RF communication.

Some of the benefits of this wireless network topology include:
a scalable solution that is based on at least a three endpoint RF communication system (Leader node-Follower node-off-body device), where each of the endpoints can initiate data exchange;
creating a robust RF channel between on-body device and off body device that relies on at least two RF radios (=Leader + Follower node) at different positions while using only one RF radio at a given time;
Leader and Follower WBAN nodes are equipped with both RF transceiver and BAN transceiver, and are located at different positions e.g. at opposite location on the body;
all WBAN nodes are connected to the Leader node, for communicating with off-body devices;
the Follower node can dynamically take over the RF communication from the Leader node when needed to ensure a backup RF channel when the situation mandates this (e.g. lost of RF connection at Leader node, low RF signal quality at Leader), thereby becoming the new Leader node; and
minimizing an amount of RF connections from WBAN to off-body device.

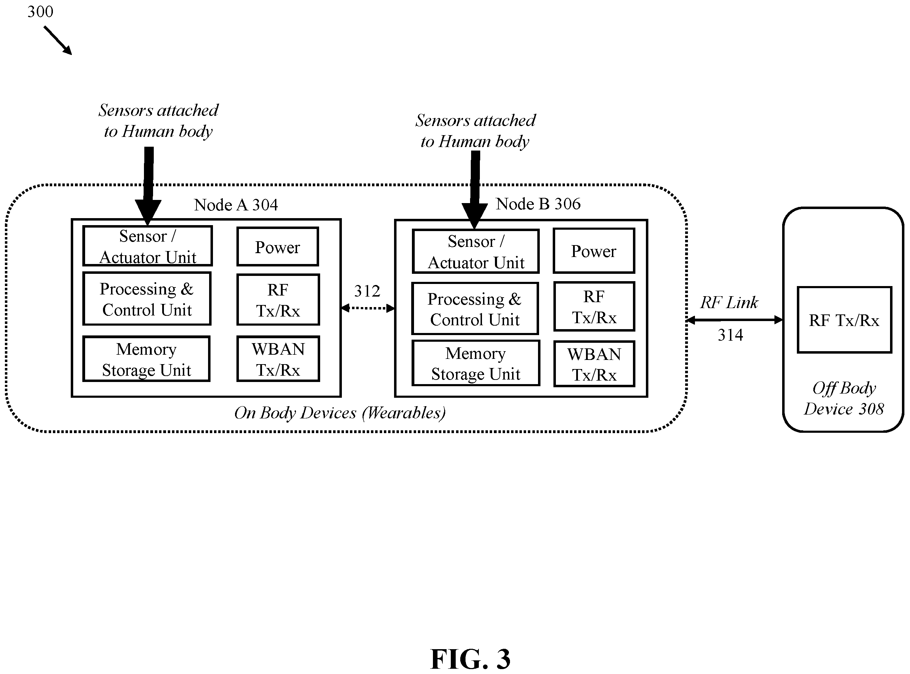

Figure 3 represents a second example 300 of the wireless network topology. The second example 300 wireless network topology shows two on-body wireless devices 304, 306 that serve as the main nodes (e.g. Node A and Node B) and an off body device 308. The two on-body wireless devices 304, 306 are configured to form a WBAN network 312 with each other, and a WPAN network 314 with the off-body wireless device 308.

An RF Tx/Rx block in each 304, 306 includes an RF Transceiver like BLE for setting-up a wireless communication channel between these on-body devices 304, 306 and an off-body device 308. The on-body devices 304, 306 could be CGM patch, Heart Beat Monitor etc., and the off-body device 308 could be smartphone or another wireless device.

A WBAN Tx/Rx block in each 304, 306 includes a transceiver for setting-up a WBAN network 312 between the on-body devices via worn/physically attached to a body (not shown). The on-body devices could be CGM patch, vitals monitoring, heart beat monitor, Hearables, Smart Watch, and so on.

A Power Unit block in each 304, 306 controls the power required for operation of the device. This unit is powered by the Battery and also monitors the battery health status.

Sensors/Actuator Unit block in each 304, 306 is connected to either a set of sensors or actuators attached to the body to monitor the vitals parameter and provide the data to processing unit. In the case of CGM patch, namely the sensor frontend circuitry that is connected to the electrochemical sensors that determine the glucose level of the patient. This sensed data is provided to the Processing Unit for further processing.

A Storage Unit block in each 304, 306 stores the processed data from Processing unit and also the sensed data from the Sensor Unit.

A Processing & Control Unit block in each 304, 306 controls the complete electronics in the on-body devices 304, 306. It activates the entire system when the wake up trigger even is received. It takes care of sensor data processing, data storage and/or actuator control. It takes care of the sequencing associated to the wireless communication (RF and/or WBAN) and takes care of data packetization and depacketization process.

The off body device 308 can be any device equipped with a far-field RF transceiver like BLE for setting up the channel for transfer of data. The device 308 could be a smart phone, tablet, Wi-Fi Hub, and so on.

Now discussed are sets of instructions for effecting the wireless network topology described above, including 1) an Initialization process, 2) an Exchange of data between on-body- and off body devices, and 3) a Checking connection possibility between on-body- and off-body devices.

In the discussion below, a Leader node is a main node that maintains the active RF line between on-body device and off body device. Other on-body nodes are communicating to this Leader node via the WBAN link, and communicate to the off body device via the RF link between Leader node and off body device. A Follower node is a main node that is capable to having an active RF line between on-body device and off body device - however, this RF link is *not* active during regular application use-case. In case of issues with the RF-link related to the Leader node, the Follower node is establishing the RF link with the off body device. When successful, the Follower node becomes the new Leader node and the "current" Leader node becomes the "new" Follower node.

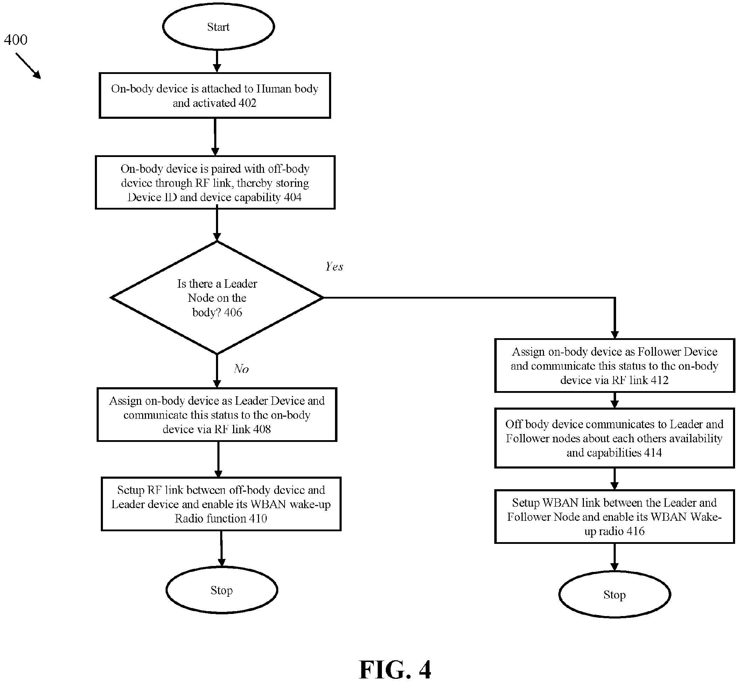

Figure 4 represents an example 400 set of instructions for a wireless network topology initialization process. As a first step, the on-body device is attached to human body and the device is activated 402. Let us consider the scenario of Node A, to be paired with an off-body device. A RF link is set-up between on-body and off body device to initiate the pairing process 404.

During this process, the Device ID and capabilities of Node A are shared with the off-body device. Capability-wise, this relates to type of device (sensing/actuator) and communication capabilities (RF and WBAN). The off body device registers the new on-body device as main node - and checks whether there are other main nodes already registered and active in the system.

When the is no other active main node in the system 406, the off body devices assigns the new node as Leader node and communicates this status to the on-body device 408, 410. When there exists already an active main node in the system, then the new node is assigned as Follower node 412. Off body device communicates to the already present Leader node about the device capability and status of the node as Follower node 414. Likewise, the Follower node also receives this information about the Leader node. The Wake-up circuitry logic of WBAN transceivers are enabled at this stage 416.

In some example embodiments, the first paired main node receives the status as Leader node. The next paired main nodes receive the status as Follower node. In an alternative embodiment a variation of such assignment is envisioned based on the quality level of the RF link, e.g. after paring a second main node, the main node with the higher-quality RF link receives the status of Leader node. Finally, the pairing process is repeated for each new main node that is applied on the human body.

Now discussed are example sets of instructions for a wireless network topology exchange of data between on-body- and off-body devices. Let us consider the scenario where two main nodes have been already paired to the off-body device i.e. Node A (Leader) and Node B (Follower) are available in the system - which means that Node A, Node B, and off body device are aware of each other. For the sake of example, let us consider an use-case application where Node A concerns an Insulin patch pump and Node B a CGM sensor patch. At any point in time, sensed data from main nodes is transferred to the off body device and actuator related data is transferred from off-body device. In some examples, e.g. artificial pancreas, sensed data from CGM device to insulin pump for actuation can also be transferred from one WBAN node to another WBAN node.

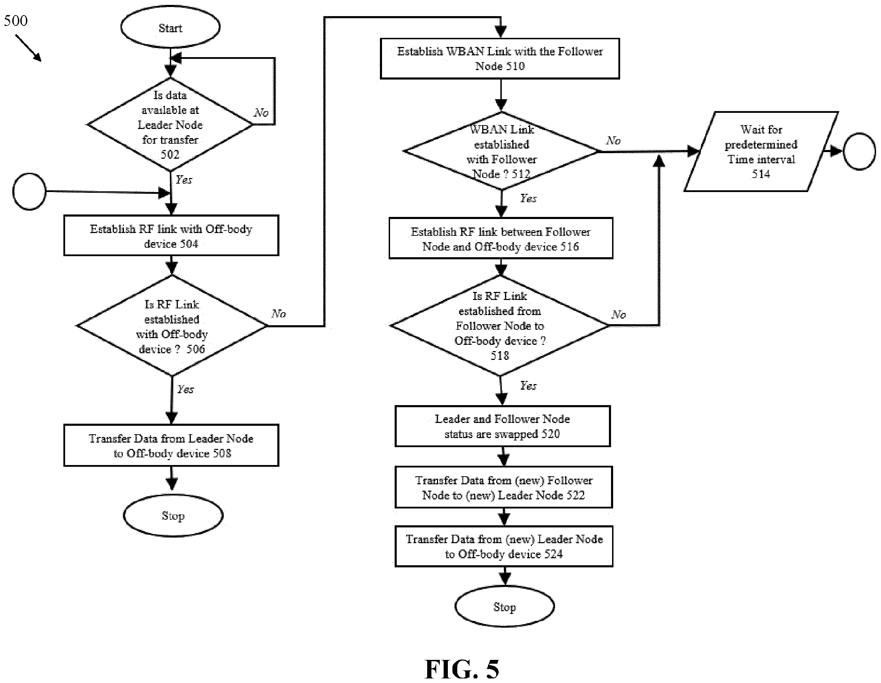

Figure 5 represents an example set of instructions 500 for a wireless network topology exchange of data between on-body and off body devices when new data is available at a Leader node 502.

The data available at the Leader node 502 is to be communicated via the RF link to the off body device 504. Once new data is available, the Leader node tries to establish an RF link with the off body device 506. When the RF link has been established successfully, the data is transmitted from Leader node to the off body device 508. In case of our example, this data could be the dose injection information from the insulin pump (Node A), transmitted to the off-body device such a user/patient can read this information within an App or the like. However, there can exist cases where the Leader node cannot manage to establish a RF link with the off-body device (e.g. when user/patient is sleeping and lies on top of the Leader node preventing setup of a RF link). In cases the RF link cannot be established, our invention proposes to handover the RF link to the follower node, which then becomes the "new" Leader node, while communicating between WBAN nodes using the WBAN transceivers 510.

When the Follower node is also not capable to establish the RF link with the off body device 512, the Leader node tries again to establish a RF link after a predefined time interval 514, and the process repeats when the RF link can't be established. However, in case when the Follower node is capable to establish the RF link 516, 518 - the data is transferred from Leader node to Follower node via the WBAN link 520, and the Follower node transmits this data 522 to the off body device 524. Note that the Follower node is now promoted as the new Leader node, while the Leader node is demoted to become the new Follower node. This status update is done at Node A, Node B and off-body device, to reflect this new system status.

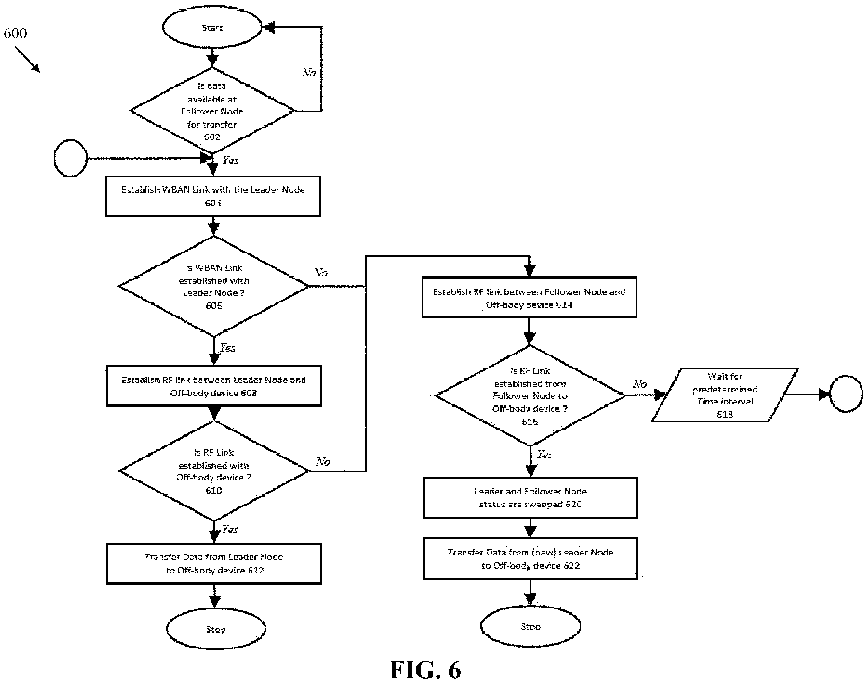

Figure 6 represents an example set of instructions 600 for a wireless network topology exchange of data between on-body and off body devices when new data is available at a Follower node 602.

A WBAN link is established with the Leader node 604, and in its turn, the Leader node tries to establish an RF link with the off-body device. When the RF link has been established successfully 606, 608, 610, the Follower node sends the data to the Leader node over the WBAN link, and this data is transmitted from Leader node to the off-body device 612. In case of our example, this data could be the sensed glucose information from the CGM patch (Node B), transmitted to Leader node (Node A, insulin pump) via the WBAN link and transmitted to the off body device (e.g. smart phone) such a user/patient can read this information within an App or the like.

When the Leader node is unable to establish the RF link with the off body device, or when the Follower node is unable to establish the WBAN link, the Follower node tries to establish the RF link with the off-body device 614. In case the Follower node is capable to establish the RF link - data is transmitted to off-body device via this channel 616, 622. The Follower node is now promoted as the new Leader node 620, while the Leader node is demoted to become the new Follower node. This status update is done at Node A, Node B and off-body device, to reflect this new system status. When the Follower node is not able to establish the RF link, after a pre-defined time interval the process is repeated.

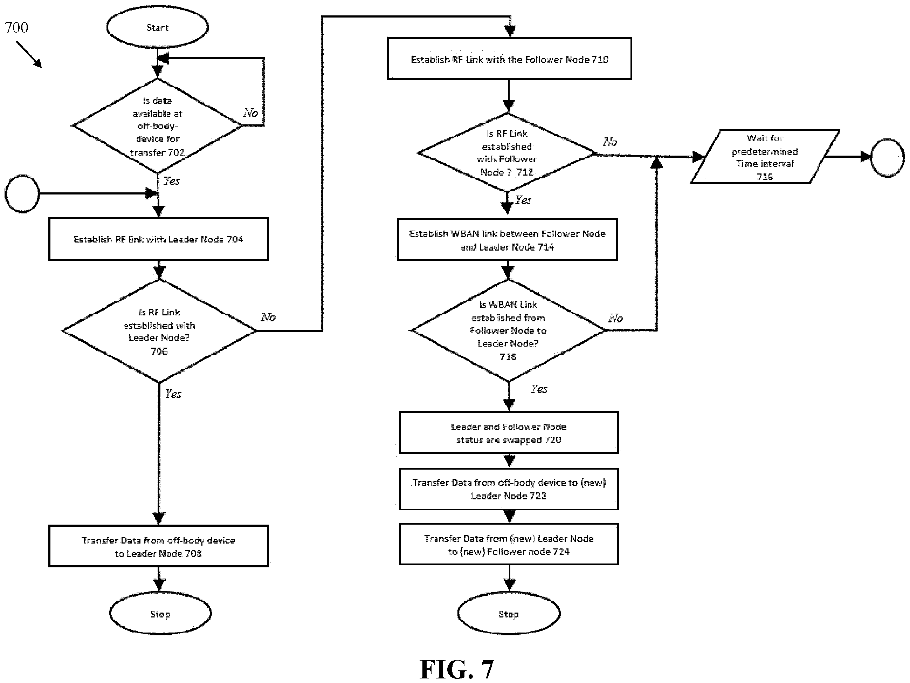

Figure 7 represents an example set of instructions 700 for a wireless network topology when new data is available at an off body device 702 to be transmitted to a Leader node, and the data is addressed to the Leader node.

Once new data is available, the off body device tries to establish an RF link with the Leader node 704, 706. When successful, the data is transmitted 708. In case of our example, this data could be to initiate a dose injection by the insulin pump (Node A). When not successful, the off-body device tries to establish an RF link with the Follower node 710, 712 - and when successful the Follower node tries to establish a WBAN link with the Leader device 714, 718.

When both are successful, the off-body device transmits the data to the Leader node via the Follower node 722, 724. The Follower node is now promoted as the new Leader node 720, while the Leader node is demoted to become the new Follower node. This status update is done at Node A, Node B and off-body device, to reflect this new system status. When no link can be established via the Follower node, after a pre-defined time interval the process is repeated. The user/patient receives a warning at the off body device that indicates the connection issue.

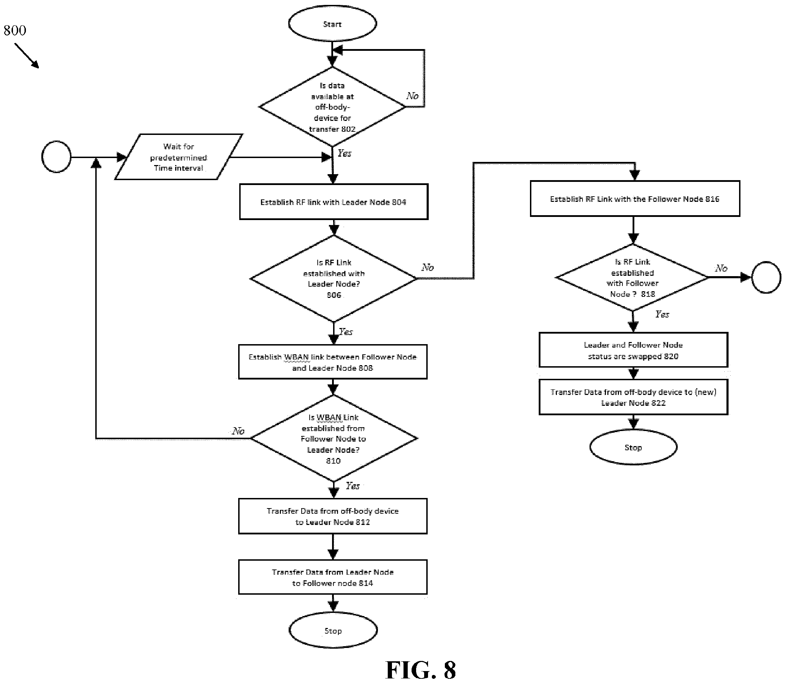

Figure 8 represents an example set of instructions 800 for a wireless network topology when new data is available at an off body device 802 to be transmitted to a Leader node, but the data is addressed to a Follower node.

Once new data is available, the off body device tries to establish an RF link with the Leader node 804, 806. When successful, the Leader node tries to establish a WBAN link with the Follower node 808, 810, and when this is also successful, the data is transmitted from off-body device to Follower node 812, 814.

When one of these links cannot be established, the off-body device tries to establish a RF link with the Follower node 816, 818. When both are successful, the off body device transmits the data to the Follower node 820, 822. The Follower node is now promoted as the new Leader node, while the Leader node is demoted to become the new Follower node. This status update is done at Node A, Node B and off body device, to reflect this new system status.

When no link can be established to the Follower node, after a pre-defined time interval the process is repeated. The user/patient receives a warning at the off-body device that indicates the connection issue.

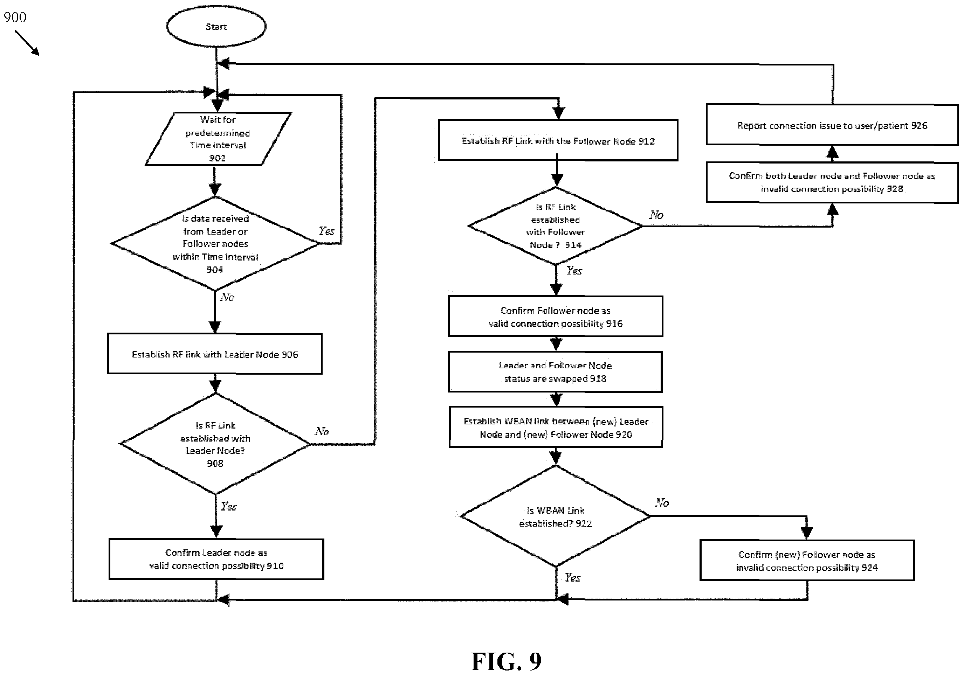

Figure 9 represents an example set of instructions 900 for checking connection validity between on-body and off body devices. In order to make the user/patient aware about any connection issues via RF link or WBAN link, we anticipate that there is a need for a connection status check (e.g. send a ping/polling signal) at a regular time interval, for warning the user/patient in case of any connection issues.

After a pre-defined time interval (e.g. 1 hour) 902 after the last RF connection between off-body device and Leader node, the off-body device checks whether an RF connection can be established 904, 906, 908. When not the case, the off body device tries to establish a connection with the Follower node 912, 914. When successful 916, the Follower node checks whether a WBAN link can be established with Leader node 920, 922, to check its presence.

When present, the Follower node is now promoted as the new Leader node 918, while the Leader node is demoted to become the new Follower node. This status update is done at Node A, Node B and off body device, to reflect this new system status. When no presence 924, the same happens while communicated to the off-body device that the "earlier" Leader node could not be found. The off body device reports the connection status to the user/patient 926, 928- and provides warnings when applicable.

In alternate example embodiments, the on-body devices are equipped with a haptic device for providing warnings to a user through vibration within the body worn device of an valid connection. In other example embodiments, the validity check can be done by Leader and Follower nodes themselves, while providing warning the user/patient through vibration.

In various other example embodiments, the on-body WBAN network can be integrated/extended with additional body-worn devices/nodes.

Option-1: Extended with WBAN nodes that contain both RF and WBAN transceivers. For this case, every next WBAN node is considered (and treated) as a Follower node. For communicating via RF link to the off-body device, the proposed WBAN setup in a star-fashion i.e. multi-point WBAN nodes (= Follower nodes) to single-point WBAN/RF node (= Leader node) from which the RF connection is managed as described before. The above discussed instructions may be modified so that Follower nodes are checked in sequence e.g. there shall a predefined list which Follower node is tried next. In essence, every Follower node has the potential to become a Leader node.

Option-2: Extended with WBAN nodes that contain a WBAN transceiver only. Like the previous option, every next WBAN node also communicates to the Leader node and operates in a star-fashion based WBAN setup. Difference is that these WBAN nodes will never become a Leader node, while never be used as Follower node in the same manner as option-1 (i.e. they are never used to connect to an off-body device due to the lacking RF transceiver). In fact, these WBAN nodes will only operate in a follower node fashion.

For pairing, an additional WBAN node sets up a WBAN link with the Leader node and sends a pairing request. The Leader node adds the device ID and capabilities of this new WBAN node, and communicates this through the system (e.g. Follower Node and off-body device).

In some example embodiments, a pairing packet structure between on-body and off-body devices includes: a Header having a Start of packet symbol, a Packet type, a Length of packet, a Type of payload information, and a Message ID. Address information can include: a Source ID, a Leader/Follower ID, and a Destination ID. Payload content can include: Data, Sensed data from on-body node + optional timestamp, Control data from off body device, and a CRC.

The header contains information about the type of packet and its content. It contains at least the following information: (a) start-of-packet symbol, (b) packet type, (c) length of packet, (d) type of payload data e.g. sensor data, control data, and (e) a message identifier.

The address section contains information about source and destination, i.e. who transmitted and who is the receiving party. The source information is especially relevant when the data is transmitted for a given WBAN node other than Leader node. Also the related Leader or Follower ID is provided or used in the address section, to identify/determine which main node transmits/receives the packet.

The payload section contains information depending on the type of packet. When a WBAN node sends sensed data to the off-body device, the payload contains the sensed data, optionally time-stamped when some level of time accuracy is needed. An example is CGM data. When the off body device sends data to a WBAN node, this concerns control data, e.g. injection dose settings for an insulin pump.

The CRC check is added to verify message integrity at receiver side.

An example system control packet may include: a Header having a Start of packet symbol, a Packet type, a Length of packet, a Message ID, and Payload content. Also included is a List of data containing overview of available WBAN nodes having a Leader ID, a Follower ID(s), Off body device ID, Capability of each node: WBAN transceiver, RF transceiver, and a CRC.

The system control packet is shared within the system during pairing process, or in case the Leader/Follower is to be swapped. The system control packet is preferably provided by the off body device to both Leader and Follower nodes. The header of the packet is similar to the data packet. The payload content concerns the list of available WBAN nodes and their capability, such Off body device, Leader- and Follower(s) are in-sync who has what role in the system at the given point in time. The CRC check is added to verify message integrity at receiver side.

In other example embodiments, a node with higher power level (i.e. remaining battery capacity) is defined as Leader Node and the other(s) as the Follower Node. During the pairing process, the assignment of Leader and Follower node is done based on the Power Level of the nodes (i.e. remaining battery capacity).

During application, battery-level status information is provided from the main nodes to the off-body device. The Follower node provides its battery-level status to the off body device via the Leader node only when data is to be shared from Follower node. Regular polling of the Follower node for battery-level is avoided in order to save power, i.e. every data communication is draining the battery.

The Leader node provides battery-level status information to the off-body device for each transmission that is performed. This information is to be added to the data packet structure for the second embodiment.

The off body device has the overview available of the communicated battery-level status information of Leader and Follower nodes, as per last communication from that node. In case there is no such information available for a given node, or when information is to be refreshed, the off body device sends the request to the given node. This request can be handled by using the data packet structure that is shown above (refer to first embodiment).

The off body device evaluates the battery-level status information for the main nodes during application. When the Power Level of the Leader node becomes lower than a given threshold as compared to the Power Level of (one of) the Follower node(s), the Follower node (with the highest Power Level) is assigned as the new Leader node.

The wireless network topology and wireless network devices discussed above have broad application, including: Medical applications, Diabetes care wearables & artificial pancreas, In-patient monitoring (vital sign monitoring inside hospital), Remote patient monitoring (vital sign monitoring outside hospital), Assisted living (measuring patient's physiological outside hospital for providing continuous medical support), Telemedicine (equipment that facilitate online consultation with doctor), Continuous Glucose Monitoring (CGM) Sensors, Connected Insulin Pumps, and Smart Insulin Pens.

The wireless network topology and wireless network devices discussed above also have applications in Non-medical applications such as: Sports (monitor the physiological activities of the wearer like heart rate, temperature, respiration rate, blood pressure, activity, and posture of any athlete in sports), Tactical (e.g. firefighters, military), Wearable audio streaming (audio from phone/watch to headphones/earbuds), Industry 4.0 (e.g. monitor machinery/equipment/assembly lines/robots/inventory and communicate to central unit/hub), Internet-of Things (IoT), and Edge devices.

One example discloses a first wireless network device, including: a far-field transceiver; a near-field transceiver; wherein the first wireless network device is configured to be wirelessly near-field coupled to a second wireless network device that also includes a far-field transceiver and a near-field transceiver; wherein the first wireless network device is further configured to be wirelessly near-field coupled to a set of near-field wireless network devices using only the near-field transceiver; wherein first wireless network device, the second wireless network device, and the set of near-field wireless network devices are configured to be in physical contact with a body; and wherein the first wireless network device is configured to be wirelessly far-field coupled to a third wireless network device only if the second wireless network device is not wirelessly far-field coupled to the third wireless network device.

In some example embodiments the set of instructions described above are implemented as functional and software instructions. In other embodiments, the instructions can be implemented either using logic gates, application specific chips, firmware, as well as other hardware forms.

When the instructions are embodied as a set of executable instructions in a non-transitory computer-readable or computer-usable media which are effected on a computer or machine programmed with and controlled by said executable instructions. Said instructions are loaded for execution on a processor (such as one or more CPUs). Said processor includes microprocessors, microcontrollers, processor modules or subsystems (including one or more microprocessors or microcontrollers), or other control or computing devices. A processor can refer to a single component or to plural components. Said computer-readable or computer-usable storage medium or media is (are) considered to be part of an article (or article of manufacture). An article or article of manufacture can refer to any manufactured single component or multiple components. The non-transitory machine or computer-usable media or mediums as defined herein excludes signals, but such media or mediums may be capable of receiving and processing information from signals and/or other transitory mediums.

It will be readily understood that the components of the embodiments as generally described herein and illustrated in the appended figures could be arranged and designed in a wide variety of different configurations. Thus, the detailed description of various embodiments, as represented in the figures, is not intended to limit the scope of the present disclosure, but is merely representative of various embodiments. While the various aspects of the embodiments are presented in drawings, the drawings are not necessarily drawn to scale unless specifically indicated.

The present invention may be embodied in other specific forms without departing from its spirit or essential characteristics. The described embodiments are to be considered in all respects only as illustrative and not restrictive. The scope of the invention is, therefore, indicated by the appended claims rather than by this detailed description. All changes which come within the meaning and range of equivalency of the claims are to be embraced within their scope.

Reference throughout this specification to features, advantages, or similar language does not imply that all of the features and advantages that may be realized with the present invention should be or are in any single embodiment of the invention. Rather, language referring to the features and advantages is understood to mean that a specific feature, advantage, or characteristic described in connection with an embodiment is included in at least one embodiment of the present invention. Thus, discussions of the features and advantages, and similar language, throughout this specification may, but do not necessarily, refer to the same embodiment.

Furthermore, the described features, advantages, and characteristics of the invention may be combined in any suitable manner in one or more embodiments. One skilled in the relevant art will recognize, in light of the description herein, that the invention can be practiced without one or more of the specific features or advantages of a particular embodiment. In other instances, additional features and advantages may be recognized in certain embodiments that may not be present in all embodiments of the invention.

Reference throughout this specification to "one embodiment," "an embodiment," or similar language means that a particular feature, structure, or characteristic described in connection with the indicated embodiment is included in at least one embodiment of the present invention. Thus, the phrases "in one embodiment," "in an embodiment," and similar language throughout this specification may, but do not necessarily, all refer to the same embodiment.

## Claims

1. A first wireless network device, comprising:
a far-field transceiver;
a near-field transceiver;
wherein the first wireless network device is configured to be wirelessly near-field coupled to a second wireless network device that also includes a far-field transceiver and a near-field transceiver;
wherein the first wireless network device is further configured to be wirelessly near-field coupled to a set of near-field wireless network devices using only the near-field transceiver;
wherein first wireless network device, the second wireless network device, and the set of near-field wireless network devices are configured to be in physical contact with a body; and
wherein the first wireless network device is configured to be wirelessly far-field coupled to a third wireless network device only if the second wireless network device is not wirelessly far-field coupled to the third wireless network device.

2. The device of claim 1:
wherein the first wireless network device, the second wireless network device, and the set of near-field wireless network devices together form a wireless near-field body area network (WBAN) according to an IEEE 802.15.6 standard.

3. The device of any preceding claim :
wherein the first wireless network device, the second wireless network device, and the third wireless network device together form a wireless far-field personal area network (WPAN) according to an IEEE 802.15.4 standard.

4. The device of any preceding claim:
wherein if the first wireless network device is far-field coupled to the third wireless network device, then the second wireless network device is blocked from being far-field coupled to the third wireless network device.

5. The device of any preceding claim:
wherein the third wireless network device is far-field coupled to only one of the wireless network devices that are in physical contact with the body.

6. The device of any preceding claim:
wherein only one of either the first wireless network device and the second wireless network device is far-field coupled to the third wireless network device at a time.

7. The device of any preceding claim:
wherein the third wireless network device communicates with all on-body devices using only the far-field coupling with either the first wireless network device or the second wireless network device.

8. The device of any preceding claim:
wherein the far-field coupling uses plane wave RF signals; and
wherein the near-field coupling uses quasi-static electric signals.

9. The device of any preceding claim:
wherein the first and second wireless network devices are on-body devices;
wherein the third wireless network devices is an off body device;
wherein if the first wireless network device is wirelessly far-field coupled to the third wireless network device then the first wireless network device is a Leader device; and
wherein if the second wireless network device is not wirelessly far-field coupled to the third wireless network device then the second wireless network device is a Follower device.

10. The device of claim 9:
wherein either the on-body Leader device, the on-body Follower device, or the off-body device is configured to switch the far-field coupling from the Leader device to the Follower device in response to a trigger condition.

11. The device of claim 10:
wherein the trigger condition occurs if the Leader device is not able to far-field couple with the off-body device.

12. The device of claim 10 or 11:
wherein the trigger condition occurs if the Leader device consumes more power to wirelessly couple to the off-body device than the Follower device.

13. The device of any of claims 10 to 12:
wherein the trigger condition occurs if the Leader device has a lower battery energy level than the Follower device.

14. The device of any of claims 10 to 13:
wherein the trigger condition occurs if the Leader device has a lower remaining battery capacity than the Follower device.

15. The device of any of claims 10 to 14:
wherein the trigger condition occurs if the Leader device at least one of reaches an end-of life, fails, or is disabled.
